(19)

Europäisches
Patentamt
European
Patent Office
Office européen
des brevets

(11) **EP 2 544 124 A1**

(12) **EUROPEAN PATENT APPLICATION**

(43) Date of publication:
**09.01.2013 Bulletin 2013/02**

(21) Application number: **11382224.1**

(22) Date of filing: **04.07.2011**

(51) Int Cl.:
*G06K 9/00* (2006.01)   *G06F 19/00* (2011.01)
*G06F 19/24* (2011.01)   *G06N 3/02* (2006.01)
*A61B 5/021* (2006.01)   *A61B 5/1455* (2006.01)
*A61B 5/145* (2006.01)   *G06N 3/00* (2006.01)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(71) Applicant: **Sabirmedical, S.L.**
**08028 Barcelona (ES)**

(72) Inventors:
• **Ribas Ripoll, Vicente Jorge**
**08030 Barcelona (ES)**
• **Wojdel, Anna**
**08028 Barcelona (ES)**

(74) Representative: **ZBM Patents**
**Zea, Barlocci & Markvardsen**
**Plaza Catalunya, 1**
**08002 Barcelona (ES)**

(54) **Methods and systems for non-invasive measurement of glucose levels**

(57)    Methods and systems for determining a glucose level for a patient in a non-invasive manner are disclosed. A photoplethysmograph (PPG) signal is obtained from a patient's measurement location. Clinical parameters of the patient are also received. Based on measurement parameters extracted from the PPG signal and the clinical parameters, a fixed length vector is generated. The fixed length vector is analyzed using a deep belief network, and an estimated glucose reading is output.

FIG. 1A

**EP 2 544 124 A1**

**Description**

**FIELD OF THE INVENTION**

Embodiments relate to a non-invasive measurement of glucose levels by analyzing photoplethysmographic (PPG) pulses.

**BACKGROUND**

Diabetes Mellitus (DM) is a group of metabolic disorders evidenced by a hyperglycemic phenotype, or high blood glucose levels in patients. Several types of DM exist, which are a result of a complex interaction between patient genetic factors, environmental factors, and lifestyle. Depending on the causes of DM, factors that may contribute to hyperglycemia may include the reduction of insulin secretion, insufficient use of glucose at the metabolic level, or an increased production of glucose by the body.

Disorders associated with DM seriously compromise the body. Thus, such disorders are a major economic burden to the healthcare system. In developed countries, DM is the primary cause of kidney failure, non-traumatic amputation of the lower extremities, and blindness in adults. Studies have shown that approximately 1.7% of the world's population suffers from DM, and that this percentage is likely to increase in the medium and long term. Thus, DM remains a major cause of morbidity and mortality.

Diagnostic criteria have been defined and published by the World Health Organization (WHO). For DM to be diagnosed, a patient typically must have a glucose concentration in a random sample of blood greater than 11 mmol/L or 200 mg/dL. Alternatively, the patient's blood plasma glucose, after fasting, typically must be above 7.0 mmol/L or 126mg/dL. A second alternative criteria diagnoses DM in a patient if the patient's blood plasma glucose two hours after consuming a liquid with dissolved glucose is greater than 11.1 mmol/L or 200 mg/dL.

Currently, measuring glucose levels involves taking a blood sample during any testing process. Various devices exist for determining glucose levels in diabetic patients, based on the reduction of the reagent glucose oxidase (GOX). Such devices use a small blood sample, obtained with a lancet, and deposit the sample on a small test strip impregnated with GOX. Glucose in the blood reacts with GOX, and hydrogen peroxide ($H_2O_2$) is obtained as a result. The amount of hydrogen peroxide causes a change in the impedance of the strip, which correlates with the level of glucose in the blood sample.

These systems are highly invasive, because they require patients with diabetes to puncture their fingers up to four times a day to obtain blood samples and monitor their glucose levels.

Systems exist that utilize spectroscopic techniques to measure glucose levels by determining the emission, transmission, and reflection of light through the skin and blood vessels. However, the measurements provided by these systems are adversely affected by water in the body, a low glucose concentration, and optical effects produced by the skin, and are thus unreliable. The need for a robust, reliable, fast and secure method for the determination of levels of glucose in blood, and which captures the metabolic complexity of glucose levels without imposing any *a priori* restriction over the functional level of an analyte to be estimated, remains unresolved.

**BRIEF SUMMARY OF THE INVENTION**

In one embodiment, a method of using PPG pulse shape measurements and patient statistics to determine glucose levels in a non-invasive manner is disclosed. Clinical parameters of a patient are received. Also, an electronic PPG signal is captured from a measurement location on the patient. Measurement parameters are extracted from the electronic PPG signal. Based on the clinical parameters and the measurement parameters, a fixed length vector is generated. An analysis using the fixed length vector as a seed vector is performed by a processor. The output of the analysis is a glucose level.

In another embodiment, a non-invasive apparatus for measuring a glucose level of a patient is disclosed. The apparatus includes a processor, and a storage coupled to the processor. The storage includes instructions that cause the processor to receive clinical parameters of a patient, and to receive a PPG signal captured from a patient. Further, the instructions cause the processor to extract measurement parameters from the PPG signal and generate a fixed length vector, based on the clinical parameters and measurement parameters. The instructions cause the processor to perform an analysis using the fixed length vector as a seed vector, and output the result of the analysis as an estimated glucose level.

Further embodiments, features, and advantages of the invention, as well as the structure and operation of the various embodiments of the invention are described in detail below with reference to accompanying drawings.

**BRIEF DESCRIPTION OF THE DRAWINGS/FIGURES**

FIG. 1A is a diagram of various systems for obtaining glucose readings, in accordance with embodiments.
FIG. 1B is a diagram of a pre-processing system for obtaining a glucose reading in accordance with an embodiment.
FIG. 2 is a diagram of a method for using PPG pulse shape measurements to determine the glucose level of a patient in accordance with an embodiment.
FIG. 3 is an exemplary plethysmograph wave profile obtained by using a digital pulse-oximeter.
FIG. 4 is a schematic illustration of an exemplary profile of a patient's blood pressure obtained by invasive catheterization.
FIG. 5 is a diagram of a non-invasive apparatus for measuring a glucose level of a patient.

Embodiments of the invention are described with reference to the accompanying drawings. In the drawings, like reference numbers may indicate identical or functionally similar elements. The drawing in which an element first appears is generally indicated by the left-most digit in the corresponding reference number.

**DETAILED DESCRIPTION OF THE INVENTION**

Certain embodiments relate to a method and system for monitoring glucose levels using data evaluated by a device capable of capturing plethysmographic signals, including optical, acoustic, or mechanical signals, such as a photoplethysmograph (PPG). Data obtained from the PPG is combined with patient data, such as gender, age, height, weight and other factors. Data may be linked to a pre-processing system to assist in estimation of the level of glucose in the patient. The pre-processing system may capture a number of parameters that affect a patient's glucose metabolic management.

The pre-processing system may be linked to a digital system, such as a system on a DSP or FPGA device, that approximates the glucose level of the patient. The digital system may employ one or more machine learning algorithms, such as a "random forest" or deep belief network trained using a Restricted Boltzmann Machine, to estimate the concentration of glucose and further reduce the estimation of errors in the post-processing stage.

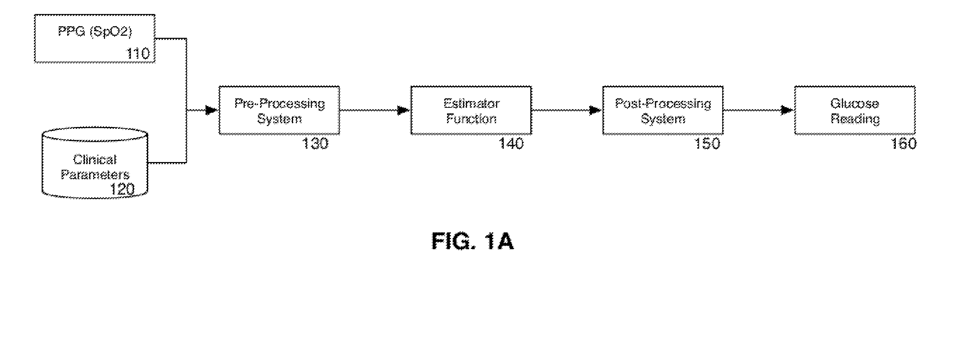

FIG. 1A is a diagram of various systems that may be utilized to estimate a patient's glucose level in accordance with an embodiment. System 110 is a PPG pulse system which may provide an SpO2 level, which will be further described below. System 120 is a system to input clinical parameters for a patient, such as height, weight, age, body mass index, and other similar clinical parameters. System 130 may be a pre-processing system to output a fixed length vector in accordance with embodiments. Estimator function system 140 may estimate a glucose level by analyzing the fixed length vector output by system 130. Further, post-processing system 150 may correct error in the values output by estimator function system 140. Glucose reading system 160 may output the glucose level of the patient in accordance with embodiments.

The information obtained by PPG pulse system 110 and clinical parameters system 120 may be processed by pre-processing system 130 to facilitate the operation of estimator function system 140. Since the PPG signal is of variable duration, a fixed dimension vector for each measurement of the PPG signal may be obtained. The vector includes information about the pulse shape (measured by autoregression coefficients and moving averages), the average distance between pulses, variance, instant energy information, energy variance, and patient medical information, such as gender, height, weight, age, body mass index, and other measures.

The estimator function system 140 may work blindly, in the sense that no functional restriction is imposed on the relationship between pulse shapes and glucose levels. Because the functional form relating the PPG pulse and the level of glucose is unknown, embodiments employ techniques to infer a robust function when presented with input variables obtained by PPG pulse system 110 and clinical parameters system 120, while ignoring irrelevant parameters derived from the PPG pulse. Embodiments may use a machine learning algorithm, such as a "random forest", deep belief network trained using restricted Boltzmann machines, or support vector machine.

In order to estimate glucose levels in accordance with certain embodiments, machine learning algorithms may require a training phase. Training may be performed only once, and no calibration or customization may be needed in the future. The training phase may include obtaining a database containing clinical information of a set of patients, including gender, weight, height, age, and other clinical parameters. Additionally, PPG pulse measurements for these patients are obtained, along with glucose measurements for the patients in the set. This information may be used to train the machine learning algorithm by minimizing errors and estimating the parameters of the machine learning algorithm.

After the training phase is complete, the parameters of the machine learning algorithm are loaded into, for example and without limitation, estimator function system 140. At that point, clinical parameters and PPG pulse shapes may be obtained from a patient having an unknown glucose measurement. This information may then be pre-processed to obtain a fixed length vector describing the PPG signal and incorporating the clinical parameters of the patient. This fixed length vector may be used by the trained machine learning algorithm to calculate the glucose level of the patient.

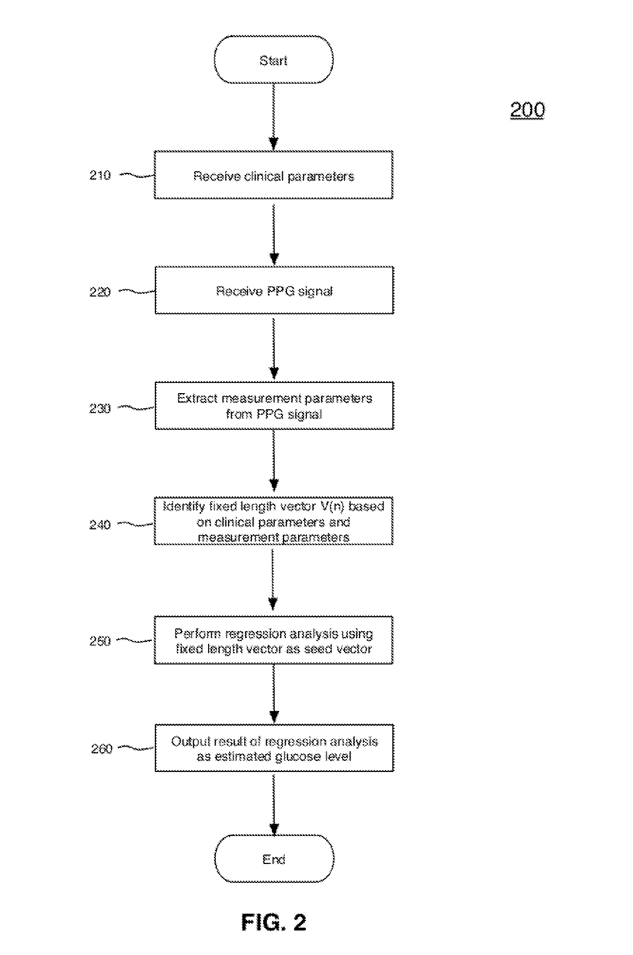

FIG. 2 is a flow diagram of a method 200 of using PPG pulse shape measurements and patient statistics to determine glucose levels in a non-invasive manner. Implementations of each step of method 100 are further described in the sections below.

At block 210, clinical parameters of a patient are received. Such clinical parameters may include height, weight, age, body mass index, and other such parameters.

At block 220, an electronic PPG signal is captured from a measurement location on a patient. The measurement location may be, for example and without limitation, a finger or an ear lobe.

At block 230, measurement parameters are extracted from the PPG signal. Measurement parameters may take into account various characteristics of the PPG signal, as will be further explained below.

Based on the measurement parameters and clinical parameters, a fixed length vector is generated at block 240. The fixed length vector may be known as V(n).

At block 250 then, an analysis, using the fixed length vector as a seed vector, is performed by a processor. The analysis may use any known regression analysis technique, such as, for example and without limitation, random forests, support vector machines, or a deep belief network trained using restricted Boltzmann machines. As will be described in further detail below, the regression analysis technique used will have been trained for a specific type of output (e.g., glucose level) using known clinical parameters, PPG signals, and output values for a training set of patients.

At block 260, the result of the analysis is output. According to an embodiment, the output value is a glucose level for a particular patient.

**Clinical Parameters**

As described with respect to block 210 of method 200, clinical parameters of a patient may be utilized to determine the glucose level of the patient. In an embodiment, these variables may include the gender, age, weight, height, and body mass index of the patient. Further, these variables may take into account whether the subject has ingested any food. Clinical parameters also may represent the current time of day. Various other variables may be received at block 210 as well, such as weight divided by age, weight divided by heart rate, height divided by heart rate, heart rate divided by age, height divided by age, age divided by body mass index, heart rate divided by body mass index, and blood pressure. These variables may be included in the generated fixed length vector V(n) generated at block 240.

**Obtaining a PPG Signal**

Obtaining a PPG curve in accordance with block 220 of method 200 may be possible using non-invasive techniques. A PPG curve may detect volume changes in a tissue's micro-vascular network. To obtain such a curve, one or more light sources may be required to illuminate the tissue where the sample is to be obtained. Additionally, one or more photodetectors may be used to measure variations in light intensity, associated with the changes in tissue perfusion in the detection volume.

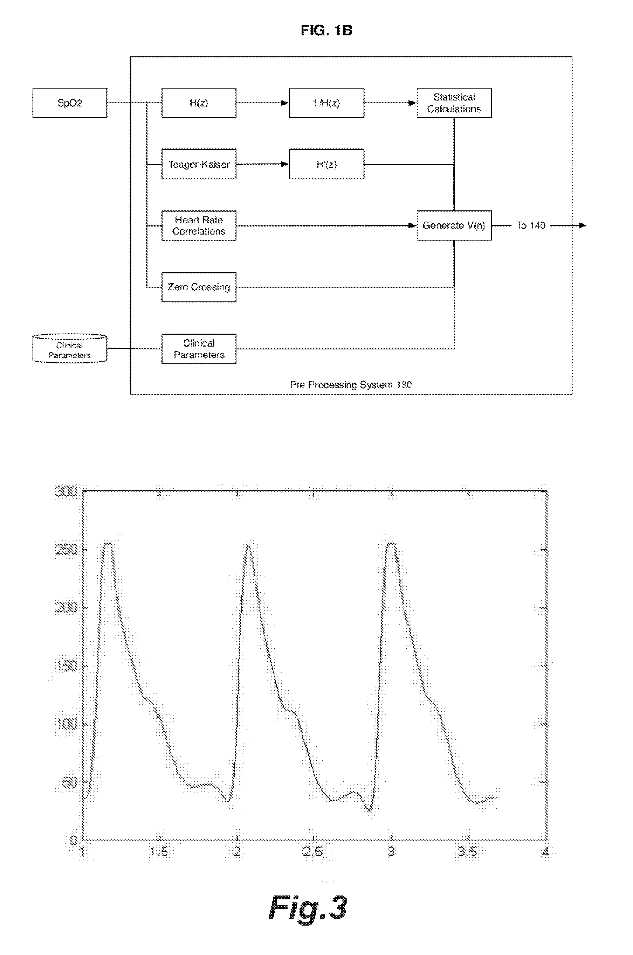

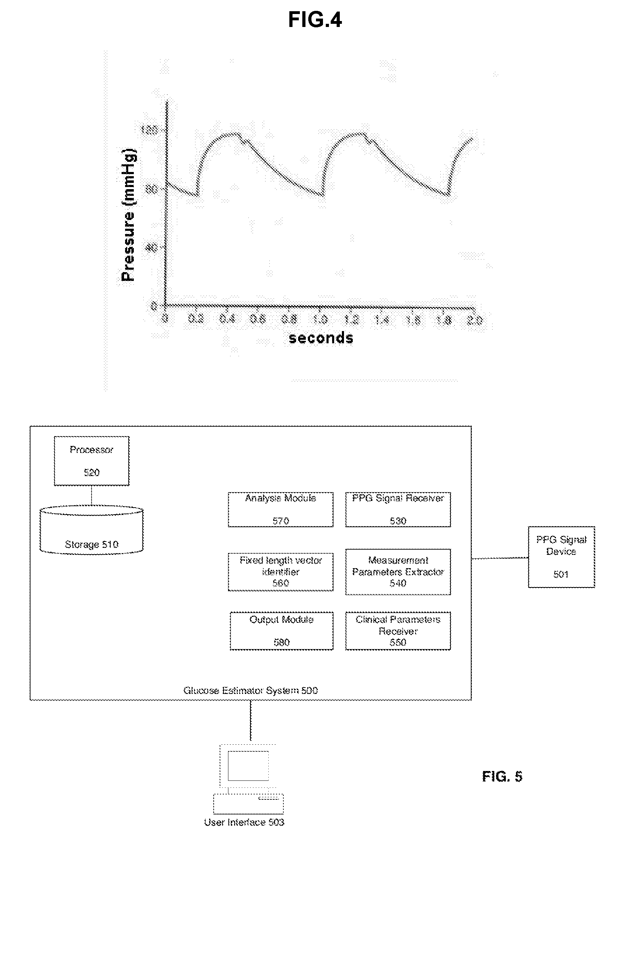

PPG detectors are commonly non-invasive, and may use infrared or near-infrared wavelengths. FIG. 3 is an exemplary PPG waveform. Peripheral pulses, as shown in FIG. 3, are most recognizable with PPG detection, and are synchronized with each heartbeat. As compared to FIG. 4, which illustrates an exemplary profile obtained by invasive catheterization, PPG detection provides similar waves to invasive catheterization.

The PPG waveform includes a physiological pulsatile waveform in the AC component, which is attributed to changes in patient blood volume synchronized to each heartbeat. This waveform overlaps with a basal component in the DC component, related to the respiratory rate, central nervous system activity, thermoregulation, and metabolic function of the patient. The fundamental frequency of the AC component may be approximately 1 Hz, depending on the patient's heart rate.

The interaction between light and biological tissues may be complex and include optical processes such as scattering, absorption, reflection, transmission, and fluorescence. The PPG wavelength used may be useful for any of the following reasons.

First, the main constituent of tissue is water. Tissue strongly absorbs light in the ultraviolet wavelengths and long wavelengths in the infrared band. In turn, there is a window in the absorption spectrum of water, which allows the passage of visible red light or near infrared light more easily through the tissue, allowing measurement of blood flow or volume at these wavelengths. Second, there are significant differences in absorption between oxyhemoglobin ($HbO_2$) and reduced hemoglobin (Hb) across most of the spectrum, except at the near infrared wavelength. Therefore, for measurements at the near-infrared wavelength (such as 805nm), the signal is not affected by changes in the oxygen saturation in the tissue.

Third, the depth of light penetration in tissue for a given radiation level may affect the desired wavelength. For PPG, the volume of penetration is approximately 1 cm^3 for transmission systems as used herein.

The PPG pulse, as seen in FIG. 3, has two distinct phases. The anacrotic, or ascending phase, may represent the increase in the patient pulse. The catacrotic phase may represent the fall of the pulse. The anacrotic phase may be related to the cardiac systole phase of the cardiac cycle, while the catacrotic phase may be related to the cardiac distole phase of the cardiac cycle. The catacrotic phase may also exhibit reflections suffered by the wave in the periphery of the circulatory system. A dicrotic pulse may also be evident in the PPG pulse. This dicrotic pulse may be found in the catacrotic phase of the PPG in healthy subjects without atherosclerosis or arterial rigidity.

**Extracting Measurement Parameters from a PPG Signal**

In an embodiment, in accordance with block 230 of method 200, various parameters may be extracted from a PPG signal in order to determine the glucose level of a patient. For example, parameters may be extracted by representing the PPG signal as a stochastic auto-regressive moving average (ARMA). Parameters also may be extracted by modeling the energy of the PPG signal using the Teager-Kaiser operator, calculating the heart rate and cardiac synchrony of the PPG signal, and determining the zero crossings of the PPG signal.

As mentioned, the spread of the pressure pulse (PP) along the circulatory tree may need to be taken into account. The PP changes its shape as it moves towards the periphery of the circulatory tree, and it may be subject to amplifications or attenuations and abnormalities in shape and temporal characteristics. These changes may be due to reflections affecting the PP due to the narrowing of arteries in the periphery. The PP pulse propagation in arteries may be further complicated by a phase-distortion dependent on the frequency of the PP. To effectively deal with these issues, the PP may be represented by a stochastic auto-regressive moving average (ARMA).

FIG. 4 is a schematic illustration of a profile of a patient's blood pressure obtained by invasive catheterization. As shown in FIG. 3 and FIG. 4, the pressure pulse shares characteristics with the PPG signal. Similar changes may be observed in vascular pathologies, such as buffering due to arterial narrowing or changes in the pulsatility.

The pulse oximeter of the system to obtain the PPG signal uses the PPG signal to obtain information about oxygen saturation (SpO2) in the arteries of the patient. The oxygen saturation reading may be obtained by illuminating tissues on an area of the skin, such as the finger or ear lobe. The tissue is illuminated in the red and near-infrared wavelengths. Typically, SpO2 devices switch between red and near-infrared wavelengths for the determination of the SpO2 level. The amplitudes of both wavelengths are sensitive to changes in SpO2 due to the difference in absorption of HbO2 and Hb at these wavelengths. The oxygen saturation level may be obtained from the ratio between the amplitudes, PPG, and the AC and DC components. These components are further explained below.

The following is a derivation of equations, according to which the SpO2 level can be calculated from a PPG signal. The following equations utilize a stochastic ARMA.

In pulse oximetry, the intensity of the light (T) transmitted through the tissue is commonly referred to as a DC signal. The intensity is a function of the optical properties of tissue, such as the absorption coefficient $\mu_a$ and the scattering coefficient $\mu'_s$. Arterial pulsation produces periodic variations in the concentrations of oxy and deoxy hemoglobin, which may result in periodic variations in the absorption coefficient.

The intensity variations of the AC component of the PPG may be expressed using the following equation:

$$\mathrm{AC} = \Delta\mathrm{T} = \frac{\partial T}{\partial \mu_a}\Big|_{\mu_a,\mu'_s} \Delta\mu_a \qquad (\mathrm{I})$$

This physiological waveform may be proportional to the variation in light intensity, which in turn is a function of the absorption and scattering coefficients ($\mu_a$ and $\mu'_s$, respectively). The component $\Delta\mu_a$, representing change in the absorption coefficient, may be written as a linear variation of the concentrations of oxy and deoxy hemoglobin ($\Delta c_{ox}$ and $\Delta c_{deox}$) as follows:

$$\Delta\mu_a = \varepsilon_{ox}\Delta c_{ox} + \varepsilon_{deox}\Delta c_{deox} \qquad (\mathrm{II})$$

where $\varepsilon_{ox}$ and $\varepsilon_{deox}$ represent the extinction coefficients of the oxy and deoxy hemoglobin forms. The extinction coefficients represent the fraction of light lost as a result of scattering and absorption per unit distance in a particular environment. Based on these equations, the arterial oxygen saturation (SpO2) may be determined by:

$$SpO_2 = \frac{\Delta c_{ox}}{\Delta c_{ox} + \Delta c_{deox}} \qquad (III)$$

Thus, the expression of SpO2 as a function of the AC component may be obtained by the application of equations (I) and (III) at the red and near-infrared wavelenghts.

$$SpO_2 = \frac{1}{1 - \dfrac{x\varepsilon_{ox}(NIR) - \varepsilon_{ox}(R)}{x\varepsilon_{deox}(NIR) - \varepsilon_{deox}(R)}} \qquad (IV)$$

In equation (IV), x may be determined using the following equation:

$$x = \frac{\left.\dfrac{\partial T(NIR)}{\partial \mu_a}\right|_{\mu_a,\mu_s'}}{\left.\dfrac{\partial T(R)}{\partial \mu_a}\right|_{\mu_a,\mu_s'}} \frac{AC(R)}{AC(NIR)} \qquad (V)$$

Normalizing the AC component to the DC component may offset the low frequency effects which are unrelated to synchronous changes in the blood. Thus, the following equation is obtained.

$$R = \frac{\dfrac{AC(R)}{DC(R)}}{\dfrac{AC(NIR)}{DC(NIR)}}$$

$$10$$

Including this parameter in (IV) yields:

$$SpO_2 = \frac{1}{1 - \dfrac{kR\varepsilon_{ox}(NIR) - \varepsilon_{ox}(R)}{kR\varepsilon_{deox}(NIR) - \varepsilon_{deox}(R)}} \qquad (VI)$$

Where k is represented by the following equation:

$$k = \frac{\dfrac{\Delta T(NIR)}{DC(NIR)}}{\dfrac{\Delta T(R)}{DC(R)}}$$

In the above equation, $\Delta T(NIR)$ and $\Delta T(R)$ correspond to equation (I), evaluated at near-infrared and red wavelengths, respectively.

Although equation (VI) is an exact solution for SpO2, k cannot be evaluated. This is because $T(\mu_a,\mu'_s)$ is not available. However, k and R (red wavelengths) are functions of the optical properties of the tissue, and thus it may be possible to represent k as a function of R. Specifically, it may be possible to express k as a linear regression of the following form:

$$k = aR + b \qquad (VII)$$

The above linear regression implies a calibration factor empirically derived. Assuming a plane wave of intensity P, the absorption coefficient may be defined as:

$$dP = \mu_a P dz \qquad (VIII)$$

where dP represents the differential change in the intensity of a light beam passing through an infinitesimal dz in a homogeneous medium with an absorption coefficient of $\mu_a$. Integrating the above equation over z yields the Beer-Lambert law.

$$P = P_0 e^{\mu_a Z} \qquad (IX)$$

Assuming that $T \approx P$, equation (VII) is thus reduced to k=1.
The PPG signal obtained by the above description may be used as the excitation or input to embodiments described herein. For example, the PPG signal may be used as the excitation or input to an exemplary pre-processing system as shown in FIG. 1B, to conduct a pre-processing in order to simplify any functions to be estimated.

Various parameters influence the form and propagation of pulse pressure (PP). These parameters may include the cardiac output, heart rate, cardiac synchrony, breathing rate, and metabolic function. As described above, the pulse pressure and photoplethysmograph may be closely related. Thus, the parameters listed above may also influence the PPG signal.

**Stochastic Auto-Regressive Moving Average**

In accordance with the above, in an embodiment, stochastic ARMA(q,p) modeling may be used to estimate the glucose level. The ARMA(q,p) model may be known as an auto-regressive moving average model of order q (MA) and p(AR).

The time series PPG(n), PPG(n-1),..., PPG(n-M) represents the realization of an AR process of order p=M, provided it satisfies the following finite difference equation (FDE):

$$PPG(n) + a_1 PPG(n-1) + \cdots + a_M PPG(n-M) = w(n) \qquad (X)$$

In the above equation, the coefficients $[a_1, a_2,..., a_M]$ are the AR parameters and $w(n)$ is a white noise or process. The term $a_k PPG(n-k)$ is the inner product of the $a_k$ coefficient and PPG(n-k), where k=1, .., M.

The above equation (X) may also be rewritten as the following:

$$PPG(n) = v_1 PPG(n-1) + v_2 PPG(n-2) + \cdots + v_M PPG(n-M) + w(n) \qquad \text{(XI)}$$

where $v_k = -a_k$.

From the above equations, it follows that the current pulse value PPG(n) equals a finite linear combination of the above values (PPG(n-k)) plus a prediction error term $w(n)$. Therefore, after rewriting the equation (X) as a linear convolution, we obtain the following equation:

$$\sum_{k=0}^{M} a_k PPG(n-k) = w(n) \qquad \text{(XII)}$$

It can be defined that $a_0$=1 without loss of generality. Thus, the Z-transform of the predictive filter may be given by:

$$A(z) = \sum_{n=0}^{M} a_n z^{-n} \qquad \text{(XIII)}$$

Defining PPG(z) as the Z-transform of the PPG pulse, then:

$$A(z)PPG(z) = W(z) \qquad \text{(XIV)}$$

where

$$W(z) = \sum_{n=0}^{M} v(n) z^{-n} \qquad \text{(XV)}$$

The moving average (MA) component of order q=K of the PPG(n) pulse may be described as the response of a linear discrete filter, or a filter with all zeros, excited by a Gaussian white noise. Thus, the MA response of the linear discrete filter written as a finite difference equation may be:

$$PPG_{MA}(n) = e(n) + b_1 e(n-1) + \cdots + b_K e(n-K) \qquad \text{(XVI)}$$

In the above equation, $[b_1, b_2, \cdots, b_K]$ are the constants called MA parameters and $e(n)$ is a white noise process of zero mean and variance $\sigma^2$. Therefore, relating equations (XII) and (XVI) we obtain the following:

$$PPG(n) = e(n) + \sum_{k=0}^{p} a_k PPG(n-k) + \sum_{k=0}^{q} b_k e(n-k) \qquad \text{(XVII)}$$

In the above equation, e(n) represents error terms of the ARMA(q,p) model. Taking the Z transform of the above equation results in the following:

$$\text{PPG}(z) = \frac{1+B(z)}{1-A(z)} E(z) \qquad \text{(XVIII)}$$

Since the first terms of the AR and MA vectors may be equal to 1 without loss of generality, the expression of the ARMA(q,p) model for the digital processing system may be given by:

$$H(z) = \frac{\overset{15}{B(z)}}{A(z)} \qquad \text{(XIX)}$$

In the above, A(z) is the AR component of PPG(n), and B(z) is the MA component of PPG(n). In an embodiment, the ARMA model is of order q=1 and p=5. In a further embodiment, an order of p and q in a range between 4 and 12 may be used. The various above operations may be known as the Wold decomposition and the Levinson-Durbin recursion.

Having calculated the ARMA(q,p) model as above, the input PPG signal is filtered with an H(z) inverse filter of FIG. 1B. The statistics of the residual error terms e(n) are calculated by a further subsystem of FIG. 1B. The results of these subsystems may be stored in an output vector V(n) of fixed dimension in accordance with block 240 of method 200.

**Teager-Kaiser Operator**

In an embodiment, the pre-processing system further contains a subsystem which calculates the Teager-Kaiser operator. The Teager-Kaiser operator provides a way to measure the energy of an oscillating signal, such as a PPG signal. The Teager-Kaiser operator may assist in measuring the energy of a system as an average sum of its squared magnitudes. Further, the pre-processing system may model the Teager-Kaiser operator's output through a p-order AR process, similar to that explained above.

In order to calculate the Teager-Kaiser operator, the PPG pulse may be considered as a signal modulated in both amplitude and frequency, or an AM-FM signal. Such a signal may be of the type represented by the equation below:

$$\text{PPG}(t) = a(t)\cos \int_0^t w(\tau(\tau) \qquad \text{(XX)}$$

In the above equation, a(t) represents the instantaneous amplitude of the PPG, and w(t) represents the instantaneous frequency of the PPG.

The Teager-Kaiser operator of the given signal may be defined by:

$$\Psi[x(t)] = [x'(t)]^2 - x(t)x''(t) \qquad \text{(XXI)}$$

$$x'(t) = \frac{dx(t)}{dt}$$

where.

The Teager-Kaiser operator applied to the AM-FM modulated signal of equation (XX) may result in the instantaneous energy of the source that produces the oscillation of the PPG. For example, the instantaneous energy may be determined by the following equation:

$$\Psi[\mathrm{PPG}(t)] \approx a^2(t)w^2(t) \qquad\qquad (\mathrm{XXII})$$

In the above, an approximation error may be negligible if the instantaneous amplitude a(t) and the instantaneous frequency w(t) do not vary too fast with respect to the average value of w(t), as is the case with the PPG pulse for the estimation of glucose levels.

The AR process of order p of the Teager-Kaiser operator, $\Psi[\mathrm{PPG}(t)]$, is implemented with a H(z) filter similar to that of FIG. 1B, as explained above. In an embodiment, the AR model is of order p=5. In a further embodiment, any order of p between 4 and 12 may be used. The result of the Teager-Kaiser operator as described herein may be stored in the fixed length vector V(n) in accordance with block 240 of method 200.

### Heart Rate and Cardiac Synchrony

Once the stochastic models based on an ARMA(q,p) model and the ARMA(q,p) model over the Teager-Kaiser operator are calculated, the heart rate and cardiac synchrony, or heart rate variability, may be calculated from the PPG signal. These variables may be determined by a further subsystem of FIG. 1B. In an embodiment, the heart rate is determined over time windows of PPG signals between 2 seconds and 5 minutes. The heart rate and cardiac synchrony variables may be stored in the fixed length vector V(n) in accordance with block 240 of method 100.

### Zero Crossings

The pre-processing system of FIG. 1B may also include a subsystem which calculates the zero crossings of the PPG signal input, as well as the variances of these zero crossings. As above, in an embodiment, the heart rate is calculated over time windows of PPG signals, between 2 seconds and 5 minutes.

All data obtained and determined by the various subsystems of FIG. 1B may be stored in the fixed dimension output vector V(n) in accordance with block 240 of method 200.

### Determining Glucose Levels

Once the fixed dimension output vector V(n) has been generated at block 240 of method 200, the estimation of the variable of interest, such as glucose level, may be determined using a function approximation system 140 of FIG. 1A, in accordance with block 250 of method 200. Such a function approximation system may utilize a regression classification algorithm, such as random forests, a deep belief network trained using restricted Boltzmann machines, a support vector machine, or any other similar regression classification algorithm. Such an algorithm used may have been trained for a specific type of output (e.g., glucose level) using known clinical parameters, PPG signals, and output values for a training set of patients.

### Random Forests

In an embodiment, a random forest regression technique is used to estimate the glucose level using the fixed length vector V(n). A random forest is a classifier consisting of a set of tree-structured classifiers, $\{h(V, \Theta_k), \mathrm{k} = 1, \cdots\}$ wherein $\Theta_k$ are random vectors independently and identically distributed, where each vector deposits a single vote for the most popular class of the input vector V. Using a random forest for estimating glucose levels may provide an advantage in that it requires no calibration after the random forest has been properly trained.

Random forests may present a reliability advantage over other classifiers based on a single tree. In addition, random forests may not impose any functional restrictions on the relationship between the pulse and glucose levels. As mentioned above, a machine learning algorithm such as random forests may first require training. Such training may include growing decision trees.

Random forests may be generated by growing decision trees based on the random vector $\Theta_k$, such that the predictor $h(V, \Theta)$ takes numeric values. The random vector $\Theta_k$ associated with each tree may provide a random distribution on each node. At the same time, the random vector also provides information on the random sampling of the training base, resulting in different data subsets for each tree.

Based on the result of the random distribution, in embodiments, the generalization error of the classifier may be provided by the following equation:

$$PE = E_{V,Y}(Y - h(V))^2 \qquad (XXIII)$$

Since the generalization error of a random forest is smaller than the generalization error of a single decision tree, defining

$$Y - h(V, \Theta) \qquad (XXIV)$$

$$Y - h(V, \Theta')$$

yields

$$PE(forest) \leq \rho PE(tree) \qquad (XXV)$$

Each decision tree may have a different generalization error. The coefficient $\rho$ represents the correlation between the residuals defined by equation (XXIV). Thus, a lower correlation between residuals may result in better estimates. In accordance with embodiments, the minimum correlation may be determined by the random sampling process of the feature vector at each node of the tree that is being trained by the pre-processing system.
To further decrease the generalization error, embodiments may estimate the variable of interest (in this case, glucose levels) and combinations of the variable of interest with parameters detailed above, such as height, weight, gender, etc.

Certain embodiments use random forests, which are a set of CART-type decision trees, or Classification and Regression Trees. These decision trees are altered to introduce systematic errors (XXV) on each tree. Further, through a system of bootstrapping, systematic variations are also introduced. Both systematic errors and systematic variations may be modeled by the parameter $\Theta_k$ in the analysis of the predictor $h(V, \Theta_k)$. Systematic error in each run may be introduced by two mechanisms. First, a random choice at each node in a subset of attributes may be used. As a result, it may not be possible to establish a statistical equivalence among the partitions made between similar nodes in different trees such that each tree behaves differently. Second, the trees may be allowed to grow up. In this case, trees act similarly to a lookup table, based on rules. Because of the sampling of the attributes, these are lookup tables to search for different structures.

The results of the above processes may be that each tree has a different systematic error.

In addition to the above modifications, each tree is trained with "bootstrap" type samples. For example, a sample of input data may be used as a bootstrap type sample, leading to a fraction of the input data being missing while another fraction of the data is duplicated. A sample of input data may include clinical parameters for a set of patients, PPG samples for that set of patients, and glucose levels for the set of patients. Glucose levels may be obtained using other known methods. The effect of the bootstrap samples is that variability is introduced. This variability may be cancelled out when estimates are averaged.

The overall result of the above techniques may be implemented by a post-processing system 150 of FIG. 1A which compensates for systematic error and variability in the error. Thus, a glucose reading 160 may represent the output of the post-processing system 150. Embodiments thus may provide a result which is more accurate than other types of function estimators (XXV). The base classifier may be a tree, which decides on the basis of levels. Thus, the system is robust when presented with input distributions which include outliers or heterogeneous data types, as in embodiments disclosed herein. Embodiments may involve taking random samples of two elements at nodes at the 47-level, with a bootstrap sample size of 100. Embodiments may also vary between two elements and 47 elements, or a bootstrap sample of between 25 and 500.

**Restricted Boltzmann Machine**

In an embodiment, one or more Restricted Boltzmann Machines (RBMs) are used to create a deep belief network (DBN) to estimate the glucose level.

A deep belief network is a stochastic generative model, consisting of multiple layers of stochastic hidden units interconnected by a set of weights. Weights are trained based on obtained data until the network performs a desired task. Using a deep belief network may allow for overcoming difficulties in training. Typically, training neural networks with multiple hidden layers requires incremental adjustment of weights based on training data. However, depending on the initial set of interconnecting weights, the learning algorithm may optimize weights to a local minimum instead of finding a global minimum. In neural networks with many hidden layers, training algorithms may be unusable.

In deep belief networks, there is an efficient, unsupervised learning process that optimizes initial weights, one layer at a time. The process may be later followed by another supervised learning procedure for fine tuning weights to train the network for specific performance. Weights for all layers in a deep belief network created by restricted Boltzmann machines (DBN-RBM) may be obtained by minimizing error as measured by the Kullback-Leibler Divergence, or approximations of the Kullback-Leibler Divergence, such as the Contrastive Divergence. However, this approach may only be suitable for classification problems or a classification analysis. Thus, in a regression analysis, Mean Squared Error may be used in place of the Kullback-Leibler Divergence or Contrastive Divergence. The Mean Squared Error of an estimator may quantify the difference between the value implied by an estimator such as a DBN-RBM, and the true value of the quantity being estimated, such as glucose.

Thus, in order to estimate glucose, a deep belief network is used which may contain one or more hidden layers. In an embodiment, three hidden layers are used. As stated above, a machine learning algorithm such as a deep belief network may first require training. Training the deep belief network may require two main steps.

In accordance with an embodiment, in the first step, each layer is trained as a separate restricted Boltzmann machine. A restricted Boltzmann machine is a two-layer neural network that consists of one layer of visible units and one layer of hidden units. Every visible unit is connected to every hidden unit with undirected weights. Further, there are no connections between visible units, and no connections between hidden units. Visible units may correspond to fixed length data vectors V(n) obtained using embodiments disclosed herein. Hidden layers may correspond to latent units used to determine glucose. Continuing the training process, the deep belief network is divided in such a way that at each consecutive layer, units from the previous layer can be treated as visible units if the restricted Boltzmann machines are trained in a particular order. For example, the RBMs may be trained starting with the input units (vectors V(n)) to the last hidden layer.

In each single RBM, the visible layer may have real-valued linear units with Gaussian noise. Latent units have a logistic distribution. As explained above, after learning one layer of hidden units, the states of the hidden units may be used as training data for the next layer of hidden units. Further, in order to reduce noise in the learning signal, binary states of latent units in the first layer may be replaced by the activation probabilities of the latent units, when being used as training data.

Training of each restricted Boltzmann machine may be done according to the following procedure.

First, probabilities of hidden units are calculated according to the following:

$$p(h|v,\Theta) = \frac{1}{1+\exp(-\sum v_i w_{ij} - b_j)}$$

where $\Theta$ is the set of network weights (w) and network biases (b). $v_i$ is the value of a visible unit, $w_{ij}$ is the weight between units i and j, and $b_j$ is a bias of a hidden unit. These probabilities are then used to calculate the expectation vale of the product of the data vector and hidden unit activation $<v_i h_j>$.

Second, each unit j is set to 1, according to the probability calculated in the first step. Using the binary states of the hidden units, the original data may be reconstructed according to the following:

$$v'_i = p(v_i)$$

where

$$p(v|h,\Theta) = \sum h_j w_{ij} + b_i$$

for the first restricted Boltzmann machine, and

$$v'_i = \frac{1}{1+\exp(-p(v_i))}$$

for the remaining restricted Boltzmann machines.

Then, probabilities of the hidden units are calculated to represent features of the reconstructed data vector, analogous to the first step. Using these updated probabilities, the expectation value of the product of the reconstructed data vector and updated probabilities is represented as $< v'_i h_j >$.

Finally, the weights in the network are changed as the basis of difference between the products obtained in the first and third step. Thus, the change in weights $w_{ij}$ may be expressed as $\Delta w_{ij} = (<v_i h_j > - <v'_i h_j >)$.

After all RBMs are trained and all hidden layers have an initial set of weights, a single unit is added representing a desired output. The initial weights of this single unit may be set to random values, and the bias of the unit may be set to the mean value of the target output data.

In accordance with an embodiment, in the second step, the constructed network is fine-tuned using error propagation. Using the constructed deep belief network, with initial weights as described above, error derivatives are backpropagated to fine-tune the network. This fine-tuning may be implemented by evaluating the error of the network's output on a sample of training data. Training data may include PPG readings, clinical parameters, and glucose values of a set of patients. The derivative of the error is calculated with respect to the output unit's weights, and minimized using the conjugate gradient method. Weights of hidden layers are then adjusted in the same manner, but using the product of error and outgoing weights as an estimate for their error vector. The procedure is repeated, starting from the output layer and progressing to the input layer, until error estimates are obtained for all hidden layers. Weights may then be iteratively optimized for each batch of training data. In this iterative step, the network's output may slowly converge towards the target output, minimizing any error. Weights may be optimized until a predefined criteria of convergence is reached.

Thus, in an embodiment, a deep belief network constructed as described herein may be used to estimate the glucose level. Such a deep belief network may be implemented in a post-processing system such as post-processing system 150 of FIG. 1A to output glucose reading 160. Using such a trained deep belief network, the fixed length data vector V (n) may be represented as a visible layer unit, and a glucose level may be the output of the deep belief network.

**Support Vector Machine**

In an embodiment, a support vector machine (SVM) may be used to estimate glucose levels. Support vector machines are a set of related supervised learning methods used for classification and regression. In a simple example, given a set of training examples, each marked as belonging to one of two categories, an SVM training algorithm may build a model that predicts whether an example not in the training examples falls into one category or the other. Thus, an SVM model may be considered as a representation of examples as points in space, such that the examples of the separate categories are divided by a gap that is as wide as possible. New examples may be mapped into the same space and predicted to belong to a category based on which side of the gap they fall on.

As an extension, a support vector machine may construct a hyperplane or set of hyperplanes in a high dimensional or infinite dimensional space, which may be used for classification or regression. An optimal support vector machine may achieve good separation by a hyperplane that has the largest distance to the nearest training datapoints of any class, since the larger the margin, the lower the generalization error or the classifier.

Thus, according to embodiments, classification may be performed by a support vector machine algorithm. A support vector machine may be implemented in a post-processing system such as post-processing system 150 of FIG.

1A to output glucose reading 160.

Thus, in accordance with the above-described embodiments, a set of known PPG values, clinical parameters, and glucose readings from a training set of patients may be used to train a regression algorithm prior to use on patients with unknown glucose levels. Then, a PPG value and clinical parameters for a patient with an unknown glucose level may be input into a sufficiently trained regression algorithm to estimate the glucose level of that patient.

FIG. 5 is a diagram of a glucose estimator system 500 that may be used to implement embodiments described herein. Glucose estimator system 500 may be coupled to PPG signal device 501 and user interface 503. PPG signal device 501 may be a device intended to capture a PPG signal from a measurement location on a patient in a non-invasive manner. For example, PPG signal device may capture a signal from a patient's finger or ear lobe.

User interface 503 may be implemented on a computing device, such as a desktop computer, laptop computer, tablet device, mobile telephone, or any other device suitable for display of information and entry of information. User interface 503 may provide clinical parameters, in accordance with embodiments, to system 500.

System 500 may be implemented using a processor 520 and a storage 510. Processor 520 may be a special purpose or general purpose processor capable of executing instructions provided by storage 510. Processor 520 may be connected to storage 510 over a bus or communications network.

Storage 510 may be a main memory, such as random access memory (RAM), or secondary memory, such as a hard disk drive. Storage 510 may also be flash memory, an optical disk drive, or a removable memory chip.

System 500 may include PPG signal receiver 530. PPG signal receiver 530 may receive a PPG signal from device 501, in accordance with embodiments. Further, system 500 may include measurement parameter extractor 540 to extract measurement parameters from the received PPG signal, in accordance with embodiments.

System 500 may also include a clinical parameters receiver 550, that receives clinical parameters for a patient from user interface 503.

System 500 may also include fixed length vector generator 560. Fixed length vector generator 560 may receive data from measurement parameters extractor 540 and clinical parameters receiver 550 to generate a fixed length vector V(n) in accordance with embodiments. Further, system 500 may include analysis module 570, which uses data provided by the fixed length vector generator 560 to perform an analysis, using a deep belief network, support vector machine, or random forest technique, to determine a glucose level, in accordance with embodiments. The analysis module may have been pre-trained, in accordance with embodiments.

Further, system 500 may include output module 580 which outputs a glucose level provided by analysis module 570 to user interface 503.

**Conclusion**

Embodiments may incorporate a display for displaying data and commands for controlling the operation of the device. In addition, embodiments may include acoustic, mechanical, and/or optical probes. The signals from any of these probes may be interpreted by a post-processing system. Such a system may be implemented using a processor and memory. Embodiments may use a digital signal processor (DSP) or a field-programmable gate array (FPGA). Memory may include, but is not limited to, RAM, hard disk, flash memory, or other memory.

Embodiments also may include manual control buttons on a handheld device. Such buttons may be used to activate and control the various systems and subsystems described herein. The handheld device may be battery powered or it may be connected to an external power source.

Results obtained by embodiments may be transmitted to a computer via serial port, USB, network connection, or other manner of coupling devices.

It is to be appreciated that the Detailed Description section, and not the Summary and Abstract sections, is intended to be used to interpret the claims. The Summary and Abstract sections may set forth one or more but not all exemplary embodiments of the present invention as contemplated by the inventor(s), and thus, are not intended to limit the present invention and the appended claims in any way.

Embodiments of the present invention have been described above with the aid of functional building blocks illustrating the implementation of specified functions and relationships thereof. The boundaries of these functional building blocks have been arbitrarily defined herein for the convenience of the description. Alternate boundaries can be defined so long as the specified functions and relationships thereof are appropriately performed.

The foregoing description of the specific embodiments will so fully reveal the general nature of the invention that others can, by applying knowledge within the skill of the art, readily modify and/or adapt for various applications such specific embodiments, without undue experimentation, without departing from the general concept of the present invention. Therefore, such adaptations and modifications are intended to be within the meaning and range of equivalents of the disclosed embodiments, based on the teaching and guidance presented herein. It is to be understood that the phraseology or terminology herein is for the purpose of description and not of limitation, such that the terminology or phraseology of the present specification is to be interpreted by the skilled artisan in light of the teachings and guidance.

The breadth and scope of the present invention should not be limited by any of the above-described exemplary embodiments, but should be defined only in accordance with the following claims and their equivalents.

**Claims**

1. A non-invasive method for measuring a glucose level of a patient, comprising:

   receiving clinical parameters of the patient;
   receiving a photoplethysmography (PPG) signal captured from a measurement location of the patient;
   extracting measurement parameters from the PPG signal;
   generating a fixed length vector based on the clinical parameters and the measurement parameters;
   performing an analysis using the fixed length vector as a seed vector to a deep belief network; and
   outputting the result of the analysis as an estimated glucose level.

2. The method for measuring a glucose level according to claim 1, wherein the deep belief network is trained using one or more restricted Boltzmann machines.

3. The method for measuring a glucose level according to claim 2, further comprising training the deep belief network using a set of clinical parameters of a plurality of patients, a set of PPG signals from the plurality of patients, and a set of glucose level values from the plurality of patients.

4. The method for measuring a glucose level according to claim 3, wherein the analysis generates an estimated glucose level without requiring calibration after the training is complete.

5. The method for measuring a glucose level according to claim 1, wherein the clinical parameters comprise at least one of: sex, age, weight, height, health information, food consumption, time of day, body mass index, or heart rate.

6. The method for measuring a glucose level according to claim 1, wherein the measurement parameters comprise at least one of: a shape of the PPG signal, a distance between pulses of the PPG signal, a variance of the PPG signal, an energy of the PPG signal, or a change in energy of the PPG signal.

7. The method for measuring a glucose level according to claim 1, wherein extracting measurement parameters is performed by using a stochastic model of a physiology of a circulatory system.

8. The method for measuring a glucose level according to claim 7, wherein the stochastic model is of the autoregressive moving average (ARMA) type.

9. The method for measuring a glucose level according to claim 1, further comprising using an error estimation technique to finalize a value of the estimated glucose level.

10. A computer readable storage medium comprising instructions stored thereon that, when executed by a processor, cause the processor to execute a method for measuring a glucose level comprising:

    receiving clinical parameters of the patient;
    receiving a photoplethysmography (PPG) signal captured from a measurement location of the patient;
    extracting measurement parameters from the PPG signal;
    generating a fixed length vector based on the clinical parameters and the measurement parameters;
    performing an analysis using the fixed length vector as a seed vector to a deep belief network; and
    outputting the result of the analysis as an estimated glucose level.

11. The computer readable storage medium according to claim 10, wherein the deep belief network is trained using one or more restricted Boltzmann machines.

12. The computer readable storage medium according to claim 11, further comprising instructions that, when executed by the processor, cause the processor to train a deep belief network using a set of clinical parameters of a plurality of patients, a set of PPG signals from the plurality of patients, and a set of glucose level values from the plurality of patients.

**13.** The computer readable storage medium according to claim 12, wherein the analysis generates an estimated glucose level without requiring calibration after the training is complete.

**14.** The computer readable storage medium according to claim 10, wherein the clinical parameters comprise at least one of: sex, age, weight, height, health information, food consumption, time of day, body mass index, or heart rate.

**15.** The computer readable storage medium according to claim 10, wherein the measurement parameters comprise at least one of: a shape of the PPG signal, a distance between pulses of the PPG signal, a variance of the PPG signal, an energy of the PPG signal, or a change in energy of the PPG signal.

**16.** The computer readable storage medium according to claim 10, wherein extracting measurement parameters is performed by using a stochastic model of a physiology of a circulatory system.

**17.** The computer readable storage medium according to claim 16, wherein the stochastic model is of the autoregressive moving average (ARMA) type.

**18.** The computer readable storage medium according to claim 17, wherein the instructions, when executed, further cause the processor to use an error estimation technique to finalize a value of the estimated glucose level.

**19.** A non-invasive apparatus for measuring a glucose level of a patient, comprising:

a processor; and
a storage coupled to the processor, wherein the storage comprises instructions which, when executed by the processor, cause the processor to perform a method for measuring a glucose level comprising:

receiving clinical parameters of the patient;
receiving a photoplethysmography (PPG) signal captured from a measurement location of the patient;
extracting measurement parameters from the PPG signal;
generating a fixed length vector based on the clinical parameters and the measurement parameters;
performing an analysis using the fixed length vector as a seed vector to a deep belief network; and
outputting the result of the analysis as an estimated glucose level.

**20.** The apparatus for measuring a glucose level according to claim 19, further comprising a plethysmographic sensor configured to measure changes in a tissue volume in a location of a patient.

**21.** The apparatus for measuring a glucose level according to claim 19, wherein the deep belief network is trained using one or more restricted Boltzmann machines.

**22.** The apparatus for measuring a glucose level according to claim 21, wherein the instructions, when executed by the processor, additionally cause the processor to train the deep belief network using a set of clinical parameters of a plurality of patients, a set of PPG signals from the plurality of patients, and a set of glucose level values from the plurality of patients.

**23.** The apparatus for measuring a glucose level according to claim 22, wherein the deep belief network generates an estimated glucose level without requiring calibration after the training is complete.

**24.** The apparatus for measuring a glucose level according to claim 19, wherein the clinical parameters comprise at least one of: sex, age, weight, height, health information, food consumption, time of day, body mass index, or heart rate.

**25.** The apparatus for measuring a glucose level according to claim 19, wherein the measurement parameters comprise at least one of: a shape of the PPG signal, a distance between pulses of the PPG signal, a variance of the PPG signal, an energy of the PPG signal, or a change in energy of the PPG signal.

**26.** The apparatus for measuring a glucose level according to claim 19, wherein extracting measurement parameters is performed by using a stochastic model of a physiology of a circulatory system.

**27.** The apparatus for measuring a glucose level according to claim 26, wherein the stochastic model is of the autoregressive moving average (ARMA) type.

FIG. 1A

FIG. 1B

FIG.3

200

```
           ┌─────────────┐
           │    Start     │
           └─────────────┘
                 │
                 ▼
210 ─── ┌──────────────────────────────┐
        │    Receive clinical parameters │
        └──────────────────────────────┘
                 │
                 ▼
220 ─── ┌──────────────────────────────┐
        │       Receive PPG signal      │
        └──────────────────────────────┘
                 │
                 ▼
230 ─── ┌──────────────────────────────┐
        │   Extract measurement parameters │
        │        from PPG signal        │
        └──────────────────────────────┘
                 │
                 ▼
240 ─── ┌──────────────────────────────┐
        │  Identify fixed length vector V(n) based │
        │       on clinical parameters and │
        │       measurement parameters  │
        └──────────────────────────────┘
                 │
                 ▼
250 ─── ┌──────────────────────────────┐
        │  Perform regression analysis using │
        │  fixed length vector as seed vector │
        └──────────────────────────────┘
                 │
                 ▼
260 ─── ┌──────────────────────────────┐
        │  Output result of regression analysis │
        │    as estimated glucose level │
        └──────────────────────────────┘
                 │
                 ▼
           ┌─────────────┐
           │     End      │
           └─────────────┘
```

FIG. 2

20

# FIG.4

# FIG. 5

Europäisches
Patentamt
European
Patent Office
Office européen
des brevets

# EUROPEAN SEARCH REPORT

Application Number

EP 11 38 2224

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2010/052354 A1 (SABIRMEDICAL S L [ES]; ESPECIALIDADES MEDICAS MYR S L [ES]; RIBAS RIPO) 14 May 2010 (2010-05-14) * the whole document * ----- | 1-27 | INV. G06K9/00 G06F19/00 G06F19/24 G06N3/02 A61B5/021 A61B5/1455 A61B5/145 G06N3/00 |
| X | ENRIC MONTE-MORENO: "Non-invasive estimate of blood glucose and blood pressure from a photoplethysmograph by means of machine learning techniques", ARTIFICIAL INTELLIGENCE IN MEDICINE, vol. 53, no. 2, 22 June 2011 (2011-06-22), pages 127-138, XP055013563, ISSN: 0933-3657, DOI: 10.1016/j.artmed.2011.05.001 * the whole document * ----- | 1-27 | |
| A | GEOFFREY E. HINTON ET AL: "A Fast Learning Algorithm for Deep Belief Nets", NEURAL COMPUTATION, vol. 18, no. 7, 1 July 2006 (2006-07-01), pages 1527-1554, XP055013559, ISSN: 0899-7667, DOI: 10.1162/neco.2006.18.7.1527 * the whole document * ----- | 1-27 | TECHNICAL FIELDS SEARCHED (IPC) G06K G06F G06N A61B |
| A | US 2010/228694 A1 (LE ROUX NICOLAS [GB] ET AL) 9 September 2010 (2010-09-09) * the whole document * ----- | 1-27 | |
| A | WO 03/063699 A1 (SENSYS MEDICAL INC [US]) 7 August 2003 (2003-08-07) * the whole document * ----- | 1-27 | |

The present search report has been drawn up for all claims

| Place of search | Date of completion of the search | Examiner |
|---|---|---|
| Munich | 1 December 2011 | Olapinski, Michael |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 11 38 2224

This annex lists the patent family members relating to the patent documents cited in the above-mentioned European search report.
The members are as contained in the European Patent Office EDP file on
The European Patent Office is in no way liable for these particulars which are merely given for the purpose of information.

01-12-2011

| Patent document cited in search report | | Publication date | Patent family member(s) | | Publication date |
|---|---|---|---|---|---|
| WO 2010052354 | A1 | 14-05-2010 | CA | 2741820 A1 | 14-05-2010 |
| | | | CN | 102245103 A | 16-11-2011 |
| | | | EP | 2353506 A1 | 10-08-2011 |
| | | | ES | 2338624 A1 | 10-05-2010 |
| | | | KR | 20110095281 A | 24-08-2011 |
| | | | US | 2011230744 A1 | 22-09-2011 |
| | | | WO | 2010052354 A1 | 14-05-2010 |
| US 2010228694 | A1 | 09-09-2010 | NONE | | |
| WO 03063699 | A1 | 07-08-2003 | CA | 2471564 A1 | 07-08-2003 |
| | | | CN | 1622785 A | 01-06-2005 |
| | | | EP | 1467652 A1 | 20-10-2004 |
| | | | JP | 2006512931 A | 20-04-2006 |
| | | | WO | 03063699 A1 | 07-08-2003 |

EPO FORM P0459

For more details about this annex : see Official Journal of the European Patent Office, No. 12/82